(19) Europäisches Patentamt
European Patent Office
Office européen des brevets

(11) **EP 2 835 643 A1**

(12) **EUROPEAN PATENT APPLICATION**
published in accordance with Art. 153(4) EPC

(43) Date of publication:
**11.02.2015 Bulletin 2015/07**

(21) Application number: **13772362.3**

(22) Date of filing: **02.04.2013**

(51) Int Cl.:
**G01N 33/543** (2006.01)   **G01N 21/64** (2006.01)

(86) International application number:
**PCT/JP2013/060125**

(87) International publication number:
**WO 2013/151066 (10.10.2013 Gazette 2013/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **06.04.2012   JP 2012087723**

(71) Applicants:
• **Konica Minolta, Inc.**
  **Tokyo 100-7015 (JP)**

• **Adtec Inc.**
  **Oita 879-0471 (JP)**

(72) Inventors:
• **TAMURA, Tsuruki**
  **Tokyo 100-7015 (JP)**
• **TAKAYAMA, Katsuyoshi**
  **Usa-shi**
  **Oita 879-0471 (JP)**

(74) Representative: **Gille Hrabal**
  **Brucknerstrasse 20**
  **40593 Düsseldorf (DE)**

(54) **METHOD FOR DETECTING OR QUANTIFYING ANALYTE, KIT FOR DETECTING OR QUANTIFYING ANALYTE, AND TEST STRIP FOR LATERAL FLOW CHROMATOGRAPHY USED TO DETECT OR QUANTIFY ANALYTE**

(57)    To provide a method for detecting or quantifying an analyte, by which the fluorescence intensity of a fluorescence can be measured in a state where an autofluorescence from the material of a test strip is reduced or eliminated, and thereby, the sensitivity in analyte detection or quantification can be improved. The method for detecting or quantifying an analyte contained in a sample according to the present invention comprises a step of, by using the specified test strip for a lateral flow type chromatography, bringing an analyte contained in a sample into contact with a labeled ligand (1) wherein a ligand (1) that is to specifically bind to the analyte has been labeled with a phosphor (1) that is to be excited by the specified light, and the specified steps.

[Fig.1]

**Description**

TECHNICAL FIELD

The present invention relates to a method for detecting or quantifying an analyte by which an analyte contained in a sample can be detected and quantified with a high sensitivity, and a kit for detecting or quantifying an analyte, and a test strip for a lateral flow type chromatography method for detecting or quantifying an analyte, which are used in such method.

BACKGROUND ART

If a test strip for a lateral flow type chromatography method (hereinafter referred to as a "test strip") is used when detecting or quantifying an analyte contained in a sample, for example, a biological sample such as blood, urine, saliva or the like, or a food extraction sample or the like, then detection and quantification can be carried out quickly and easily with no need for any large-scale equipment. Therefore, the test strip has been widely used in various clinical examinations and assay tests, including POCT (Point Of Care Testing).

This test strip has parts as described below on a membrane such as a nitrocellulose membrane; and, as a labeling substance for it, proteins including enzymes, metal colloids such as gold colloids, colored latex particles and so on have been generally used. For example, if a phosphor is contained therein as a labeling substance, then, when carrying out analyte detection and so on, a fluorescence generated by an excitation light will be detected. In this case, an ultraviolet light of from about 200 nm to about 400 nm has been used as the excitation light.

Immunochromatography methods in which such a test strip is used are roughly classified into the so-called "one-step type" and the so-called "two-step type" according to the number of their operating procedures.

In "two-step type" immunochromatographic methods, first, an analyte (for example, an antigen) is brought into contact with a ligand that is to specifically bind to the analyte and has been labeled with a phosphor (for example, an antibody against said antigen) to form a complex thereof. Next, the formed complex is added onto the test strip as a mobile phase and allowed to develop on the membrane in accordance with the principle of chromatography, and then said complex is captured by a capturing ligand (for example, a second antibody against said antigen) at a reaction part on the membrane. Thereafter, an excitation light for the phosphor is irradiated thereon, and signals emitted from the phosphor are detected, and qualitative or quantitative analysis of the analyte in the sample is carried out.

For example, Patent Document 1 discloses one mode of the "two-step type" immunochromatographic methods, and a method for quantifying an analyte with a high sensitivity is disclosed.

On the other hand, differently from the "two-step type" immunochromatographic methods, "one-step type" immunochromatographic methods do not require the step of bringing a substance to be detected into contact with a ligand that has been labeled with a phosphor, in advance before being subjected to the test strip, and therefore the detection and quantification of the analyte can be carried out more easily as compared to the "two-step type" immunochromatographic methods.

Test strips to be used in the "one-step type" immunochromatographic methods have, as their basic configuration, a membrane, and, on the membrane and in the direction the sample containing an analyte flows (in the developing direction), a reaction part (a conjugate pad) and a detection part (a test line), and optionally further have a sample addition part, a control part (a control line) and/or an absorption pad.

First, a sample containing an analyte is added onto the sample addition part, and thereby the added sample moves (develops) from the sample addition part toward the detection part on the membrane by a capillary phenomenon in the membrane. The reaction part contains a labeled ligand that is to specifically bind to an analyte (a ligand that has been labeled with a labeling substance). Therefore, the analyte binds to the labeled ligand through a specific binding reaction such as an antigen-antibody reaction to form a complex containing the analyte and the labeled ligand.

Next, the formed complex moves to the detection part. At the detection part, a ligand that is to specifically bind to the analyte in the complex (a capturing ligand) has been fixed on the membrane. Therefore, when this complex moves to the detection part, the complex is captured at the detection part through a binding reaction between the analyte in the complex and the capturing ligand. Then, at the detection part, the presence or absence, and/or the strength, of signals of the pigmentation, fluorescence or the like by the labeling substance in the captured complex are measured, and qualitative or quantitative analysis of the analyte in the sample is carried out.

Such test strips to be used in the "one-step type" immunochromatographic methods are disclosed, for example, in Patent Documents 2 to 4.

Patent Document 2 discloses a test strip by which at least two substances to be detected (analytes) contained in a specimen (a sample) can be detected or quantified at a time. Such test strip is one "on which a member for adding a sample; a conjugate pad for an immunochromatography method on which a labeling particle for a first substance to be detected has been fixed; a membrane on which, in the direction that said labeling particle for the first substance to be detected flows, an antibody-fixed part for detecting the first substance to be detected and an antibody-fixed part for capturing the labeling particle for the first substance to be detected are sequentially provided; a conjugate pad for an immunochromatography method on which a labeling particle for a second substance to be detected has been fixed; a membrane which has an antibody-fixed part for detecting the second substance; and an absorption pad, are connected in series in the order mentioned" (claim 1 and so on).

Patent Documents 3 to 4 disclose silica nanoparticles or labeled silica nanoparticles for an immunochromatography, which can give various hues, fluorescence wavelengths and so on by changing the contained labeling substance to different labeling substances and do not turn into a dark color even if they aggregate or the like. These Patent Documents further disclose test strips for an immunochromatography method, on which a member for adding a sample; a member which has been impregnated with said silica nanoparticles or said labeled silica nanoparticles for an immunochromatography; a membrane which has an antibody-fixed part; and an absorption pad, are connected in series.

PRIOR ART DOCUMENTS

PATENT DOCUMENTS

[Patent Document 1] Japanese Unexamined Patent Application Publication (JP-A) No. 2005-522698
[Patent Document 2] Japanese Unexamined Patent Application Publication (JP-A) No. 2011-27693
[Patent Document 3] Japanese Unexamined Patent Application Publication (JP-A) No. 2008-304401
[Patent Document 4] Japanese Unexamined Patent Application Publication (JP-A) No. 2009-115822

SUMMARY OF THE INVENTION

PROBLEMS TO BE SOLVED BY THE INVENTION

However, when an ultraviolet light of from about 200 nm to about 400 nm is used as an excitation light for a phosphor, which is a labeling substance, in a method for detecting or quantifying an analyte by an immunochromatography method which uses a conventional test strip, not only a fluorescence from the labeling substance on the detection part but also a fluorescence from the material constituting the test strip such as a PET film (what we call an "autofluorescence") are generated. Consequently, in the case of using a conventional test strip, the background fluorescence intensity (signals from the parts other than the detection part) is also increased along with the fluorescence intensity at the detection part, and thereby, the signal/background ratio (S/B) is lowered. In addition, in the cases where a ligand which has been labeled with a phosphor that is to specifically bind to an analyte is included in a reaction part as in the case of the "one-step type" immunochromatography, the reaction efficiency of the analyte and the ligand labeled with the phosphor is decreased, and the signal/background ratio (S/B) is further lowered. Thus, the conventional methods for detecting or quantifying an analyte still have room for improvement on their detection sensitivity and quantitativity.

In view of this, the present inventor intensively studied to discover that, if a ligand that has been labeled with a phosphor that is to be excited by a light having a wavelength of from 600 nm to 800 nm to generate a fluorescence (a labeled ligand) is used, then the fluorescence intensity of a fluorescence can be measured in a state where an autofluorescence from the material of the test strip is reduced or eliminated, and therefore, a high signal/background ratio (S/B) can be exhibited. In addition, if the zeta potential of a ligand that has been labeled with a phosphor is -30 mV or lower, then a "one-step type" immunochromatography is attained.

Thus, objects of the present invention are to provide a method for detecting or quantifying an analyte, a kit for the detection or quantification, and a test strip for a lateral flow type chromatography for the detection or quantification, by which the fluorescence intensity of a fluorescence can be measured in a state where an autofluorescence from the material of the test strip is reduced or eliminated, and therefore, a high signal/background ratio (S/B) can be exhibited, and thereby, the sensitivity in analyte detection or quantification can be improved.

MEANS FOR SOLVING THE PROBLEMS

In order to realize at least one of the above-described objects, the method for detecting or quantifying an analyte which reflects one aspect of the present invention has the following constitution. A method for detecting or quantifying an analyte contained in a sample by using a test strip for a lateral flow type chromatography which contains a membrane and a detection part on which a capturing ligand that is to specifically bind to the analyte has been fixed on the membrane, said method for detecting or quantifying an analyte comprising the following Steps (i) to (iii):

Step (i) : a step of bringing an analyte contained in a sample into contact with a labeled ligand (1) wherein a ligand (1) that is to specifically bind to the analyte has been labeled with a phosphor (1) that is to be excited by a light having a wavelength of from 600 nm to 800 nm to generate a fluorescence,

Step (ii): a step of bringing the complex (A), formed in Step (i) and containing the analyte and the labeled ligand, into contact with a capturing ligand at said detection part, and

Step (iii) : a step of irradiating on the test strip a light having a wavelength of from 600 nm to 800 nm as an excitation light for the phosphor (1) contained in the complex (A) to generate a fluorescence from the phosphor (1), and measuring the fluorescence intensity of the fluorescence.

In addition, in order to realize at least one of the above-described objects, the kit for the detection or quantification which reflects one aspect of the present invention has the following constitution. A kit for detecting or quantifying an analyte contained in a sample by using a test strip for a lateral flow type chromatography, said kit for detecting or quantifying an analyte comprising:

a test strip for a lateral flow type chromatography which contains a membrane and a detection part on which a capturing ligand that is to specifically bind to said analyte has been fixed on the membrane, and

a detection reagent which contains a labeled ligand (1) wherein a ligand (1) that is to specifically bind to said analyte has been labeled with a phosphor (1) that is to be excited by a light having a wavelength of from 600 nm to 800 nm to generate a fluorescence.

In addition, in order to realize at least one of the above-described objects, the test strip for a lateral flow type chromatography which reflects one aspect of the present invention has the following constitution.

A test strip for a lateral flow type chromatography for detecting or quantifying an analyte contained in a sample, said test strip for a lateral flow type chromatography comprising:

a membrane; and, on the membrane and in the direction that said sample develops,

a detection part on which a capturing ligand (2) that is to specifically bind to said analyte has been fixed, and,

in a region upstream of the detection part, a reaction part which contains a labeled ligand (1) wherein a ligand (1) that is to specifically bind to said analyte has been labeled with a phosphor (1) that is to be excited by a light having a wavelength of from 600 nm to 800 nm to generate a fluorescence.

## EFFECTS OF THE INVENTION

According to the present invention, the fluorescence intensity of a fluorescence can be measured in a state where generation of an autofluorescence from the material of the test strip is reduced or in a state where no autofluorescence is generated, and therefore, a high signal/background ratio (S/B) can be exhibited, and thereby, the sensitivity in analyte detection or quantification can be improved.

## BRIEF DESCRIPTION OF THE DRAWINGS

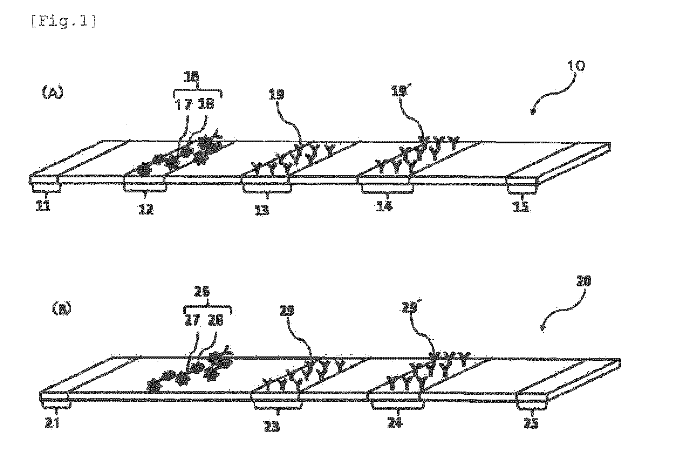

Fig. 1 (A) and (B) show modes of the test strip for a lateral flow type chromatography method which is used in the method for detecting or quantifying an analyte of the present invention.

## MODE FOR CARRYING OUT THE INVENTION

The method for detecting or quantifying an analyte according to the present invention is a method for detecting or quantifying an analyte contained in a sample by using a test strip for a lateral flow type chromatography which contains a membrane and a detection part on which a capturing ligand that is to specifically bind to the analyte has been fixed on the membrane, said method comprising the following Steps (i) to (iii) as essential steps:

Step (i) : a step of bringing an analyte contained in a sample into contact with a labeled ligand (1) wherein a ligand (1) that is to specifically bind to the analyte has been labeled with a phosphor (1) that is to be excited by a light having a wavelength of from 600 nm to 800 nm to generate a fluorescence,

Step (ii): a step of bringing the complex (A), formed in Step (i) and containing the analyte and the labeled ligand, into contact with a capturing ligand at said detection part, and

Step (iii) : a step of irradiating on the test strip a light having a wavelength of from 600 nm to 800 nm as an excitation light for the phosphor (1) contained in the complex (A) to generate a fluorescence from the phosphor (1), and measuring the fluorescence intensity of the fluorescence.

Each of these constituent features will now be described in detail.

1. Sample and Analyte

The sample is not particularly limited, as long as it contains a protein, a sugar, a nucleic acid or various types of physiologically active substance as an analyte. Examples thereof include, for example, a biological sample (i.e., whole blood, serum, plasma, urine, saliva, a sputum, a nasal cavity swab or a throat swab, spinal fluid, amniotic fluid, a nipple secretion, tears, sweat, an exudate from the skin, an extract from a tissue, cells or feces, or the like), a food extract, or the like, which contains an analyte of interest. Optionally, the analyte contained in a sample may be pretreated in advance of a step of bringing the analyte contained in the sample into contact with a labeled ligand (1) (Step (i)), in order to make specific binding reactions between the labeled ligand (1) or a capturing ligand (2) and the analyte more easily occur. Examples of the pretreatment include a chemical treatment which uses chemical agents or the like, such as an acid, a base, a surfactant and/or the like, and a physical treatment which uses heating, stirring, ultrasonic waves and/or the like, or both of them may be used in combination. In particular, in the cases where the analyte is a substance that is usually not exposed on the surface, such as an influenza virus NP antigen or the like, it is preferable to carry out a treatment with a surfactant or the like. Taking into consideration the binding reactivity between the ligand and the analyte in a specific binding reaction such as an antigen-antibody reaction or the like, as a surfactant to be used for this purpose, a nonionic surfactant may be used.

Further, the sample may be appropriately diluted with a solvent which is used in ordinary immunological analysis methods (such as water, physiological saline, a buffer solution or the like) or a water-miscible organic solvent.

Examples of the analyte include proteins such as tumor markers, signal transducers, hormones and the like (including polypeptides, oligopeptides, and the like), nucleic acids (including single-stranded or double-stranded DNAs, RNAs, polynucleotides, oligonucleotides, PNAs (peptide nucleic acids) and the like) or substances having a nucleic acid, sugars (including oligosaccharides, polysaccharides, sugar chains, and the like) or substances having a sugar chain, and other molecules such as lipids, and the analyte is not particularly limited as long as it specifically binds to a labeled ligand (1) and a capturing ligand (2). Examples thereof include, for example, carcinoembryonic antigen (CEA), HER2 protein, prostate-specific antigen (PSA), CA19-9, $\alpha$-fetoprotein (AFP), immunosuppressive acidic protein (IPA), CA15-3, CA125, estrogen receptor, progesterone receptor, fecal occult blood, troponin I, troponin T, CK-MB, CRP, human chorionic gonadotropin (HCG), luteinizing hormone (LH), follicle-stimulating hormone (FSH), syphilis antibody, influenza virus, human hemoglobin, chlamydial antigen, group A $\beta$-hemolytic streptococcal antigen, HBs antibody, HBs antigen, rotavirus, adenovirus, albumin, glycated albumin, and so on. Among these, an antigen that is solubilized by a nonionic surfactant is preferable, and an antigen that forms a self-assembly such as virus nucleoproteins is more preferable.

2. labeled ligand (1)

The labeled ligand (1) is one wherein a ligand (1) that is to specifically bind to an analyte has been labeled with a phosphor (1) that is to be excited by a light having a wavelength of from 600 nm to 800 nm to generate a fluorescence. As used herein, the term "ligand (1)" refers to an un-labeled ligand itself which is not labeled with a phosphor (1). The labeled ligand (1) is used, in Step (i) as described below, for bringing it into contact with an analyte contained in a sample to form a complex containing the analyte and the labeled ligand (1).

The phosphor (1) constituting the labeled ligand (1) is not particularly limited, as long as it is a fluorescent substance that is to be excited by a light having a wavelength of from 600 nm to 800 nm. Examples thereof include, for example, fluorescent dyes or fluorescent proteins that are to be excited by an orange visible light, a red visible light or a near infrared light. The term "fluorescent dyes" refers to fluorescent substances other than fluorescent proteins.

The light that makes a phosphor (1) emit a fluorescence (an excitation light) is a light having a wavelength of from 600 nm to 800 nm.

The fluorescence wavelength range of the fluorescence intensity which is measured at the time when the phosphor (1) emits a fluorescence (a fluorescence measurement wavelength range) is from 600 nm to 1200 nm, preferably from 650 nm to 1000 nm.

If the fluorescence measurement wavelength range is smaller than the lower limit as described above, the background fluorescence intensity may be increased in Step (iii) due to the autofluorescence from the material constituting the test strip such as PET and the detection sensitivity may be decreased. If the fluorescence measurement wavelength range is larger than the upper limit as described above, the light receiving sensitivity of the detector may be greatly decreased in Step (iii), because the quantum efficiency is decreased as the fluorescence measurement wavelength range is shifted to longer wavelengths.

Examples of a phosphor (1) which emits a fluorescence by an excitation light of from 600 nm to 800 nm include organic dyes having an indocyanine backbone such as Cy3. 5, Alixa Flor 647, Cy5, Cy5.5, Alexa Fluor 680, Cy7, Alexa Fluor 790, or the like, acridine derivatives such as acriflavine, DDAO, or the like, cyanine derivatives such as brilliant blue, brilliant green, or the like, fluorescent proteins such as allophycocyanin or the like, and so on.

The ligand (1) constituting the labeled ligand (1) is a molecule or a molecular fragment that can recognize an analyte contained in a sample or can be recognized by an analyte, and can specifically bind to the analyte.

Examples of such a molecule or such a molecular fragment include, for example, nucleic acids (DNAs, RNAs, polynucleotides, oligonucleotides, PNAs (peptide nucleic acids) and the like which may be either single-stranded or double-stranded, or nucleosides, nucleotides and modified molecules thereof), proteins (polypeptides, oligopeptides, and the like), amino acids (including modified amino acids), carbohydrates (oligosaccharides, polysaccharides, substances containing a sugar chain, and the like), lipids, or modified molecules and complexes thereof, and so on.

Examples of the protein include, for example, antibodies, lectins, and so on. In the present specification, the term "an antibody (antibodies)" includes polyclonal antibodies or monoclonal antibodies, antibodies obtained by genetic recombination, and antibody fragments.

The term "labeled" in the labeled ligand (1) means that the phosphor (1) has been directly or indirectly fixed to the labeled ligand (1) by a chemical or physical bond or adsorption, or the like, such that the phosphor (1) is not detached from the labeled ligand (1) in Steps (i) to (iii) as described below.

For example, the labeled ligand (1) may be one wherein a phosphor (1) has been directly bound to a ligand (1), or may be one wherein a ligand (1) and a phosphor (1) have been bound to each other via a linker molecule, or one wherein both of the ligand (1) and the phosphor (1) have been fixed on an insoluble particle.

The ligand (1) and the phosphor (1) are not particularly limited, as long as they have been fixed on an insoluble particle by a physical or chemical bond or adsorption, or the like, such that the phosphor (1) is not detached from the labeled ligand (1) in Steps (i) to (iii) as described below. If the insoluble particle is a particle made of a polymer compound such as a latex particle, the phosphor (1) may have been integrated into or adsorbed on the particle so as to be exposed on the surface of the particle or so as to exist near the surface of the particle such that an excitation light can reach it in Step (iii).

From the viewpoint of improving the detection sensitivity, the labeled ligand (1) is preferably one wherein a ligand (1) and a phosphor (1) have been fixed on an insoluble particle. From the viewpoint of further improving the detection sensitivity or improving the developing property on the immunostrip, it is more preferable that the average particle size of the insoluble particle be from 50 nm to 1000 nm, and it is even more preferable that said average particle size be from 100 nm to 500 nm. If said average particle size is smaller than the lower limit as described above, the intensity of the fluorescence from the phosphor (1) may be decreased in Step (iii). If said average particle size is larger than the upper limit as described above, the background fluorescence intensity may be increased in Step (iii). The average particle size is the value of an average primary particle size measured by a dynamic light scattering method.

In the cases where the labeled ligand (1) is one wherein a ligand (1) and a phosphor (1) have been fixed on an insoluble particle, the zeta potential of the labeled ligand (1) is preferably -30 mV or lower, more preferably from -51 mV to -32 mV, especially preferably from -46 mV to -35 mV. If the zeta potential is within such a range, then, when the labeled ligand (1), or a complex (A) containing the labeled ligand (1) and an analyte is subjected to the test strip, the labeled ligand (1) or the complex will not stagnate at any part and can uniformly develop. In other words, the developing property will be good. Said zeta potential is a value measured using a particle size-measuring device ("Zetasizer" manufactured by Malvern). Said zeta potential tends to move toward positive when the amount of the ligand fixed on the insoluble particle is large. Therefore, said zeta potential can be adjusted based on said ligand amount. For example, if the amount of the fixed ligand per insoluble particle (mg/g particle) is within a range of from 1 mg/g particle to 500 mg/g particle, then the zeta potential of the labeled ligand (1) will be within a suitable range of from about -60 mV to about -30 mV.

As the insoluble particle, a synthetic polymer particle, an inorganic compound particle or a polysaccharide particle is used. Examples of the synthetic polymer particle include, for example, a latex particle, a polylactide particle, and so on, but the synthetic polymer particle is not particularly limited thereto, and is preferably a latex particle. Examples of the material of the latex particle include, for example, polystyrene, styrene-butadiene copolymer, styrene-acrylate copolymer, styrene-maleic acid copolymer, polyethyleneimine, polyacrylate, polymethacrylate, polymethyl methacrylate, and so on, but the material of the latex particle is not particularly limited thereto, and is preferably polystyrene or styrene-acrylate copolymer. Examples of the inorganic compound particle include, for example, a metal particle such as gold, silver or platinum, or a metal colloid particle, a porous glass particle, a metal oxide particle such as silica or alumina, but the inorganic compound particle is not particularly limited thereto. Examples of the polysaccharide particle include, for example, an agarose particle, a dextran particle, a cellulose particle, a chitosan particle, and so on, but the polysaccharide particle is not particularly limited thereto.

Examples of the method of fixing a ligand (1) on an insoluble particle can be roughly classified into methods by a physical adsorption of a ligand (1) onto an insoluble particle, and methods by a covalent bond as a chemical bond. Examples of the former include, for example, a method in which a ligand (1) is added to a solution in which silica particles or gold particles have been colloidally dispersed, and thereafter the resultant is left to stand for a predetermined period of time to allow physical adsorption. Methods like this have the advantage that the operations are easy. On the other hand, examples of the latter include, for example, a method in which, by using a condensing agent, a carboxyl group introduced onto the particle surface of the insoluble particle and an amino group of the ligand (1) are bound to each other via an amide bond, and a method in which, by using a so-called cross-linking reagent, the insoluble particle and the ligand (1) are bound to each other. Methods like this have the advantage that the ligand (1) can be quantitatively and irreversibly introduced onto the insoluble particle. In addition, after fixing a phosphor (1) and a ligand (1) on an insoluble particle by a method as described above, it is preferable to add a blocking agent such as a bovine serum albumin solution onto them to block the particle surface on which the antibody is in an unbound state.

### 3. Test Strip for Lateral Flow Type Chromatography

The test strip for a lateral flow type chromatography (the term may be referred to simply as the test strip) contains, at least, a membrane and a detection part on which a capturing ligand that is to specifically bind to an analyte has been fixed on the membrane, and may optionally contain an optional member(s) such as a reaction part, a reagent addition part, a control part and/or a water absorption pad as described below. For example, the test strip represented by the numbering 10 of Fig. 1 (A) has, on the membrane and in the direction that the sample develops, a sample addition part 11, a reaction part 12 which contains a labeled ligand (1) 17, a detection part 13 on which a capturing ligand (2) 19 has been fixed, a control part 14 on which a capturing ligand (3) 19' has been fixed, and a water absorption pad 15. Alternatively, the test strip represented by the numbering 20 of Fig. 1 (B) has, on the membrane and in the direction that the sample develops, a sample addition part 21, a detection part 23 on which a capturing ligand (2) 29 has been fixed, a control part 24 on which a capturing ligand (3) 29' has been fixed, and , but does not have a reaction part.

### (1) Membrane

As is the case of a membrane used for a common test strip, the membrane to be used for the test strip is, for example, one formed with an inactive substance (a substance that does not react with an analyte, ligands, phosphors, and so on) made of a microporous substance, which exhibits a capillary phenomenon and in which a sample develops as soon as the sample is added onto it. Specific examples of the membrane include a fibrous or nonwoven fibrous matrix, a membrane, a filter paper, a glass fiber filter paper, cloth, cotton, and so on, which are constituted by polyurethane, polyester, polyethylene, polyvinyl chloride, polyvinylidene fluoride, nylon, a cellulose derivative such as nitrocellulose or cellulose acetate, and/or the like. Among these, a membrane, a filter paper, a glass fiber filter paper, or the like, which is constituted by a cellulose derivative or nylon, is preferably used, and a nitrocellulose membrane, a mixed nitrocellulose ester (a mixture of nitrocellulose and cellulose acetate) membrane, a nylon membrane, or a filter paper is more preferably used.

The form and size of the membrane are not particularly limited, as long as they are suitable in view of the actual operations and at the time of measuring the fluorescence intensity as described below. In order to make the operations easier, it is preferable that the membrane be supported by a support made of plastic or the like.

### (2) Detection Part

The detection part is not particularly limited, as long as it is constituted such that a complex (A) containing a labeled ligand (1) and an analyte is brought into contact with a capturing ligand (2) that is to specifically bind to the analyte. The detection part may be one wherein a capturing ligand (2) has been directly fixed on the membrane, or may be one wherein a capturing ligand (2) has been fixed on a pad made of cellulose filter paper, glass fiber, nonwoven fabric or the like, which pad has been fixed on the membrane.

In the present specification, the phrase "a capturing ligand has been fixed" means the state where the capturing ligand has been directly or indirectly immobilized on the membrane by a physical or chemical bond or adsorption, or the like so as not to move from the detection part even when a sample is supplied to the test strip.

### (3) Sample Addition Part

The test strip may have a sample addition part that is for a sample containing an analyte to be added, which is, in the cases where a reaction part has not been formed as shown in Fig. 1 (B), in a region upstream of the detection part, or, in the cases where a reaction part has been formed as shown in Fig. 1 (A), in a region upstream of the reaction part, in the direction that the sample develops.

The sample addition part is a part that is for a sample containing an analyte to be accepted into the test strip, and may be one which has been formed on the membrane, or one on which a sample addition pad, which is constituted by a material such as cellulose filter paper, glass fiber, polyurethane, polyacetate, cellulose acetate, nylon, cotton cloth, or the like, has been formed on the membrane. A sample addition part having a sample addition pad is preferable because it can exhibit a function of filtering aggregates and the like in the sample. In addition, from the viewpoint of preventing the decrease in the analytical accuracy due to the non-specific adsorption of the analyte in the sample to the material of the sample addition part, it is preferable that the material constituting the sample addition pad be treated in advance to prevent the non-specific adsorption.

### (4) Reaction Part

On the test strip, it is preferable that, as shown by the numbering 12 of Fig. 1 (A), on the membrane and in the direction that the sample flows, a reaction part containing a labeled ligand (1) have been formed in a region upstream of the detection part. If a reaction part has been formed on the test strip like this, then, when a sample containing an analyte is subjected to the reaction part or the sample addition part, the analyte contained in the sample can be brought into contact with the labeled ligand (1) at the reaction part. Therefore, it is not required to carry out, as Step (i), a step of bringing the analyte contained in the sample into contact with the labeled ligand (1), and thereafter supply the resultant to the test strip. In other words, if the sample is subjected to the reaction part or the sample addition part, then, by that alone, Step (i) can be carried out, and, as a result, a complex (A) containing the analyte and the labeled ligand (1) can be easily formed.

The reaction part is not particularly limited, as long as it contains a labeled ligand (1) that is to specifically bind to an analyte. The reaction part may be one wherein a labeled ligand (1) has been directly applied on the membrane, or may be one wherein a pad made of cellulose filter paper, glass fiber, nonwoven fabric or the like (a conjugate pad) has been impregnate with a labeled ligand (1), and the pad impregnated with the labeled ligand (1) has been fixed on the membrane.

### (5) Control Part

On the test strip, as shown by the numbering 14 of Fig. 1(A) and the numbering 24 of Fig. 1 (B), a control part on which a third ligand that is to specifically bind to a labeled ligand (1) has been fixed may have been formed on the membrane and in the direction that the sample develops. If the measurement of the fluorescence intensity is carried out not only at the detection part but also at the control part in Step (iii) as described below, then the confirmation that the sample, which had been subjected to the test strip, developed thereon and reached the reaction part and the detection part, and thereby the examination was normally carried out will become possible. Except for using a capturing ligand (3) instead of the capturing ligand (2), the control part can be made in the same manner and can take the same constitution as in the case of the detection part described above.

### (6) Water Absorption Pad

On the test strip, on the membrane and in the direction that the sample develops, a water absorption pad may have been formed, in the cases where a control part has not been formed, in a region downstream of the detection part, or, in the cases where a control part has been formed, in a region downstream of the control part as shown by the numbering 15 of Fig. 1 (A) and the numbering 25 of Fig. 1 (B).

The water absorption pad is formed, for example, from a water absorptive material such as cellulose filter paper, nonwoven fabric, cloth, cellulose acetate, or the like. The moving speed of the sample after the development front (front line) of the added sample comes to the water absorption pad varies depending on the material, the size and/or the like of the water absorption pad. Therefore, by selecting the material, the size and/or the like of the water absorption pad, the speed can be set so as to be suitable for analyte detection or quantification.

### Steps (i) to (iii)

Step (i) is a step of bringing an analyte contained in a sample into contact with a labeled ligand (1) wherein a ligand (1) that is to specifically bind to the analyte has been labeled with a phosphor (1) that is to be excited by a light having a wavelength of from 600 nm to 800 nm to generate a fluorescence. The mode of the contact therein is not particularly limited, as long as a complex (A) containing the analyte and the labeled ligand (1) is formed.

For example, the mode may be a mode in which a sample is subjected to the reaction part or the sample addition part of the test strip and thereafter Step (i) is carried out at the reaction part of the test strip, or a mode in which a sample is brought into contact with the labeled ligand (1) without using the test strip before a sample is subjected to the test strip.

In the case of the former, the test strip is required to have a reaction part, as shown by the numbering 10 of Fig. 1 (A). However, it is not required to bring an analyte contained in a sample into contact with a labeled ligand (1) and thereafter supply the resultant to the test strip, and, if the sample is subjected to the reaction part or the sample addition part, then, by that alone, a complex (A) containing the analyte and the labeled ligand (1) can be easily formed.

On the other hand, in the case of the latter, the test strip is not required to have a reaction part, as shown by the numbering 20 of Fig. 1 (B). However, it is required to bring an analyte contained in a sample into contact with a labeled ligand (1) in the detection reagent according to the present invention and thereafter supply the resultant to the test strip.

Subsequently, the complex (A) formed in Step (i) develops on the test strip and arrives at the detection part. Then, as Step (ii), a step of bringing the complex (A), formed in Step (1) and containing the analyte and the labeled ligand (1), into contact with a capturing ligand (2) at the detection part of the test strip is carried out. When the complex (A) is brought into contact with the capturing ligand (2), the capturing ligand (2) recognizes the analyte in the complex (A) or is recognized by the analyte, and specifically binds to the analyte in the complex (A). As a result, the complex (A) is captured at the detection part. If the labeled ligand (1) alone arrives at the detection part, then the sole labeled ligand (1) passes through the detection part, because the capturing ligand (2) does not specifically bind to the labeled ligand (1). In the cases where a control part has been formed, the labeled ligand (1), which passes through the detection part, continues to develop; and, when it arrives at such control part, since a capturing ligand (3) that is to specifically bind to the labeled ligand (1) has been fixed thereon, the labeled ligand (1) binds to the capturing ligand (3). As a result, the labeled ligand (1), which does not form a complex (A) with an analyte, is captured at the control part.

Further, after Step (ii) and optionally before carrying out Step (iii), a step of washing the test strip with a buffer solution widely used in biochemical examinations such as water, physiological saline, a phosphate buffer solution or the like to remove the free labeled ligand (1) which has not been captured at the detection part or at the detection part and the control part (a labeled ligand (1) which does not form a complex (A) with an analyte) (hereinafter referred to as "the washing step") may be carried out. If such step is carried, then, when the intensity of a fluorescence from a phosphor (1) at the detection part or at the detection part and the control part is measured in Step (iii), the background fluorescence intensity can be reduced, and the signal/background ratio can be increased, and the detection sensitivity and the quantitativity can be further improved.

After Step (ii) or optionally carrying out the washing step, a step of irradiating on the test strip an orange visible light, a red visible light or a near infrared light as an excitation light for the phosphor (1) contained on the labeled ligand (1) in the complex (A) to generate a fluorescence from the phosphor (1), and measuring the fluorescence intensity of the fluorescence (Step (iii)) is carried out. The material of the test strip, particularly in the case of an organic polymer, generates an autofluorescence by a light having a wavelength smaller than visible light wavelengths (for example, an ultraviolet light). However, in this step, a light having a specified long wavelength is irradiated as an excitation light for the phosphor (1). Accordingly, although an autofluorescence as described above is still generated, the intensity thereof can be decreased. Therefore, increase in the background fluorescence intensity caused by an autofluorescence can be reduced, and the signal/background ratio can be improved, and the detection sensitivity and the quantitativity can be improved.

Thus, increase in the background fluorescence intensity caused by an autofluorescence can be reduced, and the signal/background ratio can be improved, and the detection sensitivity and the quantitativity can be improved.

The excitation light varies depending on the excitation wavelength of the phosphor (1), but is a light having a wavelength of from 600 nm to 800 nm. If such an excitation light is irradiated, then, even in the cases where the test strip (for example, a membrane) is constituted by a material which generates an autofluorescence by an ultraviolet light (for example, PET), generation of the autofluorescence can be reduced or the generation can be eliminated, and therefore the analyte can be detected and quantified with a high detection sensitivity.

As a means by which the intensity of a fluorescence from a phosphor (1) is measured in Step (iii), a known apparatus for detecting fluorescence signals, such as a CCD detector, can be used optionally in combination with a filter which can cut signals having a particular wavelength.

In the cases where a control part has been formed on the test strip, by Step (ii), the labeled ligand (1) is captured by a capturing ligand (3) at the control part to form a complex containing the labeled ligand (1) and the capturing ligand (3). Therefore, if an excitation light for the phosphor (1) is irradiated on the test strip as Step (iii), then, not only at the detection part but also at the control part, emission of a fluorescence can be generated and the intensity of the fluorescence from the phosphor (1) can be measured. If the measurement of the fluorescence intensity is carried out not only at the detection part but also at the control part in this way, then, the confirmation based on the measured fluorescence intensities whether the sample, which had been subjected to the test strip, developed thereon and reached the reaction part and the detection part will become possible. In other words, if the fluorescence is not detected at the control part, this can be judged as a failure of the examination.

5. Kit for Detecting or Quantifying Analyte

As another mode of the present invention, a kit to be used in a method for detecting or quantifying an analyte contained in a sample by using the test strip for a lateral flow type chromatography as described above is provided. The kit according to the present invention comprises, as essential components, a test strip for a lateral flow type chromatography which contains a membrane and a detection part on which a capturing ligand that is to specifically bind to said analyte has been fixed on the membrane, and a detection reagent which contains a labeled ligand (1) wherein a ligand (1) that is to specifically bind to said analyte has been labeled with a phosphor (1) that is to be excited by an orange visible light, a red visible light or a near infrared light, and may optionally further comprise other component(s).

The kit according to the present invention may be used in a mode in which Step (i) is carried out by bringing an analyte in a sample into contact with a labeled ligand (1) in the detection reagent, and thereafter the sample is subjected to the reaction part or the sample addition part of the test strip to carry out Steps (ii) to (iii) sequentially. Alternatively, the kit may be used in a mode in which the detection reagent is applied to a region upstream of the detection part on the test strip and appropriately dried to form a reaction part, and thereafter, a sample is added onto the formed reaction part or onto a region upstream of the reaction part (for example, the sample addition part) to carry out Steps (i) to (iii) sequentially.

EXAMPLES

The present invention will now be described in detail by way of Examples, but the present invention is not limited to the Examples below.

[Comparative Example 1A]

[Preparation of Labeled Antibody 1A]

An anti-influenza A virus monoclonal antibody (manufactured by Millipore Corporation, Anti-Influenza A, nucleoprotein, clone A3) was dialyzed against a 50 mM MES (2-Morpholinoethanesulfonic acid, monohydrate: manufactured by DOJINDO LABORATORIES) buffer (pH 6.0) solution. Thereafter, by using a phosphor 1A (FAM (5-FAM-X (6-(fluorescein-5-carboxamido) hexanoic acid, succinimidyl ester), Kirkegaard & Perry Laboratories, Inc.) and making said monoclonal antibody and the phosphor 1A bind to each other via its amino group, a detection solution 1A containing a labeled antibody 1A was prepared.

[Production of Test Strip 1A]

An anti-influenza A virus monoclonal antibody (manufactured by Millipore Corporation, Anti-Influenza A, nucleoprotein, clone A3) was dialyzed against 10 mM Tris-HCl (pH 7.5). After the dialysis, the resultant was filtered through a filter having a pore size of 0.22 $\mu$m and diluted with 10 mM Tris-HCl (pH 7.5) to prepare a capturing antibody (2) solution containing the anti-influenza A virus monoclonal antibody.

In addition, anti-mouse IgG antibody (manufactured by Adar Biotech Ltd., Anti-IgG, Mouse, Goat-Poly) was dialyzed against 10 mM Tris-HCl (pH 7.5). After the dialysis, the resultant was filtered through a filter having a pore size of 0.22 $\mu$m and diluted with 10 mM Tris-HCl (pH 7.5) to prepare a capturing antibody (3) solution containing the anti-mouse IgG antibody.

Then, using a positive pressure spraying device (BioJet; BioDot, Inc.), the capturing antibody (2) solution and the capturing antibody (3) solution were applied onto a trinitrocellulose membrane (manufactured by Millipore Corporation, white, 60 mm width x 350 mm length) as a line at the positions 7 mm and 14 mm from the edge of the membrane in the developing direction (from said edge toward another edge), respectively. Warm air of 45°C was sprayed thereon for 10 minutes, and thereafter the membrane was dried to form a detection part and a control part, respectively. In addition, a nonwoven fabric made of polyester (6 mm width x 10 mm length) was impregnated with the detection solution 1A, and the resulting nonwoven fabric impregnated with the detection solution 1A was fixed in a region upstream of the detection part on the membrane.

Next, in order to fix the membrane and improve the strength thereof, a backing sheet made of plastic (manufactured by BioDot, Inc.) was adhered onto the opposite side surface of the membrane of the surface on which the detection solution 1A had been applied.

Next, a cellulose nonwoven fabric was cut into a size of 15 mm in width and 10 cm in length, and this was attached onto the upper surface of the membrane so as to overlap 2 mm with the upstream edge of the membrane to form a sample addition part.

In addition, a cellulose filter paper of 30 mm in width and 10 cm in length (Whatman) was attached onto the upper surface of the membrane so as to overlap 5 mm with the downstream edge of the membrane to form a sample absorption pad. Finally, the membrane was cut along the long axis direction at intervals of 5 mm to produce a test strip 1A.

[Measurement of Signal/Background Ratio (S/B)]

As an analyte, influenza A virus was added to a buffer solution (20 mM MES buffer solution (pH 6.0), 1 (W/V) % Triton X-100, 2 (W/V) % arginine hydrochloride, 1.0 (W/V) % bovine serum albumin) so as to attain 280.0 pfu/ml (pfu: plaque-forming unit), and the resultant was suspended to prepare a sample. The prepared sample was added onto the sample addition part of the test strip 1A, and said sample was allowed to develop on the test strip 1A from the sample addition part to the water absorption pad. After washing, an excitation light having a wavelength of 488 nm was irradiated on the test strip 1A by using a fluorescence measuring device, and the intensity of a fluorescence having a wavelength of 520 ± 30 nm was measured.

The fluorescence measuring device as mentioned above has a light emitting part for irradiating an excitation light which makes a phosphor (1) excite and a light receiving part for receiving emission of a fluorescence from the phosphor (1) and converting it into electrical signals, and is constituted such that the light emitting part irradiates an excitation light from an angle at which the excitation light specularly reflected by the phosphor does not come into the light receiving part.

The signal/background ratio (S/B) was calculated according to the following equation (1). The calculated signal/background ratio (S/B) is shown in Table 1.

[Equation 1]

$$S/B = [(\text{Fluorescence Intensity at Dtection Site}) - (\text{Background fluorescenc intensity})] / \text{Backgrround fluorescence intensity}$$

Herein, the "background fluorescence intensity" represents the fluorescence intensity of the whole test strip except for the detection part and the control part.

[Example 1B]

A detection solution 1B containing a labeled antibody 1B was prepared and a test strip 1B was produced in the same manner as in Comparative Example 1A except for using a fluorescent protein 1B ("allophycocyanin", manufactured by DOJINDO LABORATORIES) instead of the phosphor 1A and making said monoclonal antibody and the fluorescent protein 1B bind to each other via its thiol group.

Then, the signal/background ratio (S/B) was calculated in the same manner as in Comparative Example 1A except for using the test strip 1B instead of the test strip 1A, changing the wavelength of the excitation light from 488 nm to 633 nm, and measuring the intensity of a fluorescence having a wavelength of 660 ± 10 nm. The results are shown in Table 1.

[Example 1C]

A detection solution 1C containing a labeled antibody 1C was prepared and a test strip 1C was produced in the same manner as in Comparative Example 1A except for using a phosphor 1C (Alexa Fluor 680 (manufactured by Molecular Probes Inc.) instead of the phosphor 1A and making said monoclonal antibody and the phosphor 1C bind to each other via its carboxyl group.

Then, the signal/background ratio (S/B) was calculated in the same manner as in Comparative Example 1A except for using the test strip 1C instead of the test strip 1A, changing the wavelength of the excitation light from 488 nm to 680 nm, and measuring the intensity of a fluorescence having a wavelength of 700 ± 10 nm. The results are shown in Table 1.

[Example 1D]

A detection solution 1D containing a labeled antibody 1D was prepared and a test strip 1D was produced in the same manner as in Comparative Example 1A except for using a phosphor 1D (Alexa Fluor 780 (manufactured by Molecular Probes Inc.) instead of the phosphor 1A and making said monoclonal antibody and the phosphor 1D bind to each other via its carboxyl group.

Then, the signal/background ratio (S/B) was calculated in the same manner as in Comparative Example 1A except for using the test strip 1D instead of the test strip 1A, changing the wavelength of the excitation light from 488 nm to 780 nm, and measuring the intensity of a fluorescence having a wavelength of 800 ± 10 nm. The results are shown in Table 1.

[Table 1]

Table 1

| | | Comparative Example 1A | Example 1B | Example 1C | Example 1D |
|---|---|---|---|---|---|
| Antibody | | Anti-Influenza A Virus Monoclonal Antibody | Anti-Influenza A Virus Monoclonal Antibody | Anti-Influenza A Virus Monoclonal Antibody | Anti-Influenza A Virus Monoclonal Antibody |
| Phosphor | Type | FAM | Allophycoc vanin | Alexa Fluor 680 | Alexa Fluor 780 |
| | Maximum Excitation Wavelength (nm) | 480 | 650 | 660 | 770 |
| | Maximum Fluorescence Wavelength (nm) | 520 | 660 | 690 | 810 |
| Wavelength of Excitation Light (nm) | | 488 | 633 | 680 | 780 |
| Measurement Wavelength (nm) | | 520 ± 30 | 660 ± 10 | 700 ± 10 | 800 ± 10 |
| Signal | | 10 | 244.7 | 2648874 | 722832 |
| Background | | 52.5 | 54.7 | 262751 | 39904 |
| S/B Ratio | | 0.19 | 4.47 | 10.08 | 18.11 |

[Example 2A]

A detection solution 2A containing a labeled antibody 2A was prepared and a test strip 2A was produced in the same manner as in Comparative Example 1A except for using a phosphor 2A as described in Table 2 (Alexa Fluor 680, manufactured by Molecular Probes Inc.) instead of the phosphor 1A and making said monoclonal antibody and the phosphor 2A bind to each other.

Then, the signal/background ratio (S/B) was calculated in the same manner as in Comparative Example 1A except for using the test strip 2A instead of the test strip 1A, changing the excitation wavelength from 488 nm to 680 nm, and measuring the intensity of a fluorescence having a wavelength of 700 ± 10 nm. The results are shown in Table 2.

[Example 2B]

A detection solution 2B containing a labeled antibody 2B was prepared and a test strip 2B was produced in the same manner as in Comparative Example 1A except for using a phosphor 2B as described in Table 2 (Alexa Fluor 790, manufactured by Molecular Probes Inc.) instead of the phosphor 1A and making said monoclonal antibody and the phosphor 2B bind to each other.

Then, the signal/background ratio (S/B) was calculated in the same manner as in Example 2A except for using the test strip 2B instead of the test strip 2A. The results are shown in Table 2.

[Example 2C]

A detection solution 2C containing a labeled antibody 2C was prepared and a test strip 2A was produced in the same manner as in Comparative Example 1A except for using a fluorescent latex particle 2C as described in Table 2 (FC02F8612 (average particle size: 0.39 μm, manufactured by Bangs Laboratories, Inc.)) instead of the phosphor 1A and making said monoclonal antibody and the fluorescent latex particle 2C bind to each other via the carboxyl group of the fluorescent particle 2C.

Then, the signal/background ratio (S/B) was calculated in the same manner as in Example 2A except for using the test strip 2C instead of the test strip 2A. The results are shown in Table 2.

[Example 2D]

A detection solution 2D containing a labeled antibody 2D was prepared and a test strip 2D was produced in the same manner as in Comparative Example 1A except for using a fluorescent latex particle 2D as described in Table 2 (FC02F8782 (average particle size: 0.32 μm, manufactured by Bangs Laboratories, Inc.)) instead of the phosphor 1A and making said monoclonal antibody and the fluorescent latex particle 2D bind to each other via the carboxyl group of the fluorescent latex particle 2D.

Then, the signal/background ratio (S/B) was calculated in the same manner as in Example 2A except for using the test strip 2D instead of the test strip 2A. The results are shown in Table 2.

[Table 2]

Table 2

| | | | Example 2A | Example 2B | Example 2C | Example 2D |
|---|---|---|---|---|---|---|
| Antibody | | | Anti-Influenza A Virus Monoclonal Antibody | Anti-Influenza A Virus Monoclonal Antibody | Anti-Influenza A Virus Monoclonal Antibody | Anti-Influenza A Virus Monoclonal Antibody |
| Phosphor | Type | | Alexa Fluor 680 | Alexa Fluor 790 | FC02F8612 | FC02F8782 |
| | Maximum Excitation Wavelength (nm) | | 660 | 770 | 660 | 770 |
| | Maximum Fluorescence Wavelength (nm) | | 690 | 810 | 690 | 810 |
| Insoluble Particle | | | Not Used | Not Used | Latex Particle (Average Particle Size: 0.39 μm) | Latex Particle (Average Particle Size: 0.32 μm) |
| Wavelength of Excitation Light (nm) | | | 680 | 780 | 680 | 780 |
| Measurement Wavelength (nm) | | | 700 ± 10 | 800 ± 10 | 700 ± 10 | 800 ± 10 |
| S/B Ratio | | | 0.24 | 0.41 | 6.30 | 15.40 |

[Example 3A]

A detection solution 3A containing a labeled antibody 3A was prepared and a test strip 3A was produced in the same manner as in Comparative Example 1A except for using a fluorescent latex particle 3A as described in Table 3 (FC02F8655 (average particle size: 65 nm, manufactured by Bangs Laboratories, Inc.)) instead of the phosphor 1A and making said monoclonal antibody and the fluorescent latex particle 3A bind to each other via the carboxyl group of the fluorescent latex particle 3A.

Then, the signal/background ratio (S/B) was calculated in the same manner as in Comparative Example 1A except for using the test strip 3A instead of the test strip 1A, changing the excitation wavelength from 488 nm to 680 nm, and measuring the intensity of a fluorescence having a wavelength of 700 ± 10 nm. The results are shown in Table 3.

[Example 3B]

A detection solution 3B containing a labeled antibody 3B was prepared and a test strip 3B was produced in the same manner as in Example 3A except for using a fluorescent latex particle 3B as described in Table 3 (FC02F9770 (average particle size: 190 nm, manufactured by Bangs Laboratories, Inc.)) instead of the phosphor 1A and making said monoclonal antibody and the fluorescent latex particle 3B bind to each other via the carboxyl group of the fluorescent latex particle 3B.

Then, the signal/background ratio (S/B) was calculated in the same manner as in Example 3A except for using the test strip 3B instead of the test strip 3A. The results are shown in Table 3.

[Example 3C]

A detection solution 3C containing a labeled antibody 3C was prepared and a test strip 3C was produced in the same manner as in Example 3A except for using a fluorescent latex particle 3C as described in Table 3 (Lx (average particle size: 300 nm, manufactured by Fujikura Kasei Co., Ltd.)) instead of the phosphor 1A and making said monoclonal antibody and the fluorescent latex particle 3C bind to each other via the carboxyl group of the fluorescent latex particle 3C.

Then, the signal/background ratio (S/B) was calculated in the same manner as in Example 3A except for using the test strip 3C instead of the test strip 3A. The results are shown in Table 3.

[Example 3D]

A detection solution 3D containing a labeled antibody 3D was prepared and a test strip 3D was produced in the same manner as in Example 3A except for using a fluorescent latex particle 3D as described in Table 3 (FC02F9990 (average particle size: 400 nm, manufactured by Bangs Laboratories, Inc.)) instead of the phosphor 1A and making said monoclonal antibody and the fluorescent latex particle 3D bind to each other via the carboxyl group of the fluorescent latex particle 3D.

Then, the signal/background ratio (S/B) was calculated in the same manner as in Example 3A except for using the test strip 3D instead of the test strip 3A. The results are shown in Table 3.

[Example 3E]

A detection solution 3E containing a labeled antibody 3E was prepared and a test strip 3E was produced in the same manner as in Example 3A except for using a fluorescent latex particle 3E as described in Table 3 (FC02F8632 (average particle size: 510 nm, manufactured by Bangs Laboratories, Inc.)) instead of the phosphor 1A and making said monoclonal antibody and the fluorescent latex particle 3E bind to each other via the carboxyl group of the fluorescent latex particle 3E.

Then, the signal/background ratio (S/B) was calculated in the same manner as in Example 3A except for using the test strip 3E instead of the test strip 3A. The results are shown in Table 3.

[Example 3F]

A detection solution 3F containing a labeled antibody 3F was prepared and a test strip 3F was produced in the same manner as in Example 3A except for using a fluorescent latex particle 3F as described in Table 3 (FC02F4194 (average particle size: 890 nm, manufactured by Bangs Laboratories, Inc.)) instead of the phosphor 1A and making said monoclonal antibody and the fluorescent latex particle 3F bind to each other via the carboxyl group of the fluorescent latex particle 3F.

Then, the signal/background ratio (S/B) was calculated in the same manner as in Example 3A except for using the test strip 3F instead of the test strip 3A. The results are shown in Table 3.

[Table 3]

Table 3

| | | Example 3A | Example 3B | Example 3C | Example 3D | Example 3E | Example 3F |
|---|---|---|---|---|---|---|---|
| Antibody | | Anti-Influe nza A Virus Monoclonal Antibody | Anti-Influe nza A Virus Monoclonal Antibody | Anti-Influe nza A Virus Monoclonal Antibody | Anti-Influe nza A Virus Monoclonal Antibody | Anti-Influe nza A Virus Monoclonal Antibody | Anti-Influe nza A Virus Monoclonal Antibody |
| Phosphor | Type | FC02F8655 | FC02F9770 | Lx | FC02F9990 | FC02F8632 | FC02F4194 |
| | Maximum Excitation Wavelength (nm) | 660 | 660 | 660 | 660 | 660 | 660 |
| | Maximum Fluorescence Wavelength (nm) | 690 | 690 | 690 | 690 | 690 | 690 |
| Insoluble Particle | | Used, Average Particle Size: 65 nm | Used, Average Particle Size: 190 nm | Used, Average Particle Size: 300 nm | Used, Average Particle Size: 400 nm | Used, Average Particle Size: 510 nm | Used, Average Particle Size: 890 nm |
| Wavelength of Excitation Light (nm) | | 680 | 680 | 680 | 680 | 680 | 680 |
| Measurement Wavelength (nm) | | 700 $\pm$ 10 | 700 $\pm$ 10 | 700 $\pm$ 10 | 700 $\pm$ 10 | 700 $\pm$ 10 | 700 $\pm$ 10 |
| S/B Ratio | | 0.2 | 3.4 | 6.8 | 5.7 | 2.9 | 0.3 |

EP 2 835 643 A1

[Example 4A]

A detection solution 4A containing a labeled antibody 4A was prepared and a test strip 4A was produced in the same manner as in Comparative Example 1A except for using a fluorescent latex particle 4A as described in Table 4 (FS02F9862 (average particle size: 190 nm, manufactured by Bangs Laboratories, Inc.)) instead of the phosphor 1A and making said monoclonal antibody and the fluorescent latex particle 4A bind to each other.

In addition, the zeta potential of the labeled antibody 4A was measured using a particle size-measuring device ("Zetasizer" manufactured by Malvern.

Then, as an analyte, influenza A virus was added to a buffer solution (20 mM MES buffer solution (pH 6.0), 1 (W/V) % Triton X-100, 2 (W/V) % arginine hydrochloride, 1.0 (W/V) % bovine serum albumin) so as to attain 280.0 pfu/ml (pfu: plaque-forming unit), and the resultant was suspended to prepare a sample. The obtained sample was added onto the sample addition part of the test strip 4A, and said sample was allowed to develop from the sample addition part to the water absorption pad. After washing, an excitation light having a wavelength of 680 nm was irradiated on the test strip 1A by using a fluorescence measuring device, and a fluorescent image for a fluorescence having a wavelength of 700 ± 10 nm was obtained.

Observation of the obtained fluorescent image was carried out to evaluate the developing property in accordance with the following evaluation criteria.

[Evaluation Criteria]

Good: In the region upstream of the detection part, fluorescence was scarcely observed.

Somewhat Good: In the region upstream of the detection part, a little fluorescence was observed.

Not Good; In the region upstream of the detection part, fluorescence was significantly observed.

The greater fluorescence intensity in the region upstream of the detection part indicates worse developing property of the labeled antibody, and indicates that a larger amount of the labeled antibody does not arrive at the detection part and is stagnating in the region upstream of the detection part.

[Example 4B]

A detection solution 4B containing a labeled antibody 4B was prepared and a test strip 4B was produced and the developing property of the labeled antibody 4B was evaluated in the same manner as in Example 4A except for using a fluorescent latex particle 4B as described in Table 4 (FC02F9770 (average particle size: 190 nm, manufactured by Bangs Laboratories, Inc.)) instead of the fluorescent latex particle 4A and making said monoclonal antibody and the fluorescent latex particle 4B bind to each other.

[Example 4C]

A detection solution 4C containing a labeled antibody 4C was prepared and a test strip 4C was produced in the same manner as in Example 4A except for using a fluorescent latex particle 4C as described in Table 4 (FC02F8612 (average particle size: 390 nm, manufactured by Bangs Laboratories, Inc.)) instead of the fluorescent latex particle 4A and making said monoclonal antibody and the fluorescent latex particle 4C bind to each other. In addition, the zeta potential of the labeled antibody 4C was measured and the developing property of the labeled antibody 4C was evaluated in the same manner as in Example 4A.

[Example 4D]

A detection solution 4D containing a labeled antibody 4D was prepared and a test strip 4D was produced in the same manner as in Example 4A except for using a fluorescent latex particle 4D as described in Table 4 (FC02F9990 (average particle size: 400 nm, manufactured by Bangs Laboratories, Inc.)) instead of the fluorescent latex particle 4A and making said monoclonal antibody and the fluorescent latex particle 4D bind to each other. In addition, the zeta potential of the labeled antibody 4D was measured and the developing property of the labeled antibody 4D was evaluated in the same manner as in Example 4A.

[Example 4E]

A detection solution 4E containing a labeled antibody 4E was prepared and a test strip 4E was produced in the same manner as in Example 4A except for using a fluorescent latex particle 4E as described in Table 4 (FC02F9889 (average particle size: 490 nm, manufactured by Bangs Laboratories, Inc.)) instead of the fluorescent latex particle 4A and making said monoclonal antibody and the fluorescent latex particle 4E bind to each other. In addition, the zeta potential of the labeled antibody 4E was measured and the developing property of the labeled antibody 4E was evaluated in the same manner as in Example 4A.

[Example 4F]

A detection solution 4F containing a labeled antibody 4F was prepared and a test strip 4F was produced in the same manner as in Example 4A except for using a fluorescent latex particle 4F as described in Table 4 (FKFL1171 (average particle size: 220 nm, manufactured by Fujikura Kasei Co., Ltd.)) instead of the fluorescent latex particle 4A and making said monoclonal antibody and the fluorescent latex particle 4F bind to each other. In addition, the zeta potential of the labeled antibody 4F was measured and the developing property of the labeled antibody 4F was evaluated in the same manner as in Example 4A.

[Example 4G]

A detection solution 4G containing a labeled antibody 4G was prepared and a test strip 4G was produced in the same manner as in Example 4A except for using a fluorescent latex particle 4G as described in Table 4 (FKFL1175 (average particle size: 310 nm, manufactured by Fujikura Kasei Co., Ltd.)) instead of the fluorescent latex particle 4A and making said monoclonal antibody and the fluorescent latex particle 4G bind to each other. In addition, the zeta potential of the labeled antibody 4G was measured and the developing property of the labeled antibody 4G was evaluated in the same manner as in Example 4A.

[Example 4H]

A detection solution 4H containing a labeled antibody 4H was prepared and a test strip 4H was produced and the developing property of the labeled antibody 4H was evaluated in the same manner as in Example 4A except for using a fluorescent latex particle 4H as described in Table 4 (Lx (average particle size: 200 nm, manufactured by Fujikura Kasei Co., Ltd.)) instead of the fluorescent latex particle 4A and making said monoclonal antibody and the fluorescent latex particle 4H bind to each other.

[Example 4I]

A detection solution 4I containing a labeled antibody 4I was prepared and a test strip 4I was produced and the developing property of the labeled antibody 4I was evaluated in the same manner as in Example 4A except for using a fluorescent latex particle 4I as described in Table 4 (Lx (average particle size: 300 nm, manufactured by Fujikura Kasei Co., Ltd.)) instead of the fluorescent latex particle 4A and making said monoclonal antibody and the fluorescent latex particle 4I bind to each other.

[Table 4]

Table 4

| | | Example 4A | Example 4B | Example 4C | Example 4D | Example 4E | Example 4F | Example 4G | Example 4H | Example 4I |
|---|---|---|---|---|---|---|---|---|---|---|
| Antibody | | Anti-Influenza A Virus Monoclonal Antibody | Anti-Influenza A Virus Monoclonal Antibody | Anti-Influenza A Virus Monoclonal Antibody | Anti-Influenza A Virus Monoclonal Antibody | Anti-Influenza A Virus Monoclonal Antibody | Anti-Influenza A Virus Monoclonal Antibody | Anti-Influenza A Virus Monoclonal Antibody | Anti-Influenza A Virus Monoclonal Antibody | Anti-Influenza A Virus Monoclonal Antibody |
| Phosphor | Type | FS02F9862 | FC02F9770 | FC02F8612 | FC02F9990 | FC02F9889 | FKFL1171 | FKFL1175 | Lx | Lx |
| | Maximum Excitation Wavelength (nm) | 660 | 660 | 660 | 660 | 660 | 660 | 660 | 660 | 660 |
| | Maximum Fluorescence Wavelength (nm) | 690 | 690 | 690 | 690 | 690 | 690 | 690 | 690 | 690 |
| Average Particle Size of Insoluble Particle (nm) | | 190 | 190 | 390 | 400 | 490 | 220 | 310 | 200 | 300 |
| Zeta Potential (mV) | | -39 | -35.3 | -45.2 | -50.6 | -32.4 | -25.7 | -28.7 | -27.4 | -25.5 |
| Wavelength of Excitation Light (nm) | | 680 | 680 | 680 | 680 | 680 | 680 | 680 | 680 | 680 |
| Measurement Wavelength (nm) | | 700 ± 10 | 700 ± 10 | 700 ± 10 | 700 ± 10 | 700 ± 10 | 700 ± 10 | 700 ± 10 | 700 ± 10 | 700 ± 10 |
| Developing Property Evaluation | | Good | Good | Good | Somewhat Good | Somewhat Good | Not Good | Not Good | Not Good | Not Good |

DESCRIPTION OF SYMBOLS

| | |
|---|---|
| 10, 20: | Test Strip for Lateral Flow Type Chromatography Method |
| 11, 21: | Sample Addition Part |
| 12: | Reaction Part |
| 13, 23: | Detection Part |
| 14, 24: | Control Part |
| 15, 25: | Water Absorption Pad |
| 16, 26: | Complex (A) |
| 17, 27: | Labeled Ligand (1) |
| 18, 28: | Analyte |
| 19, 29: | Capturing Ligand (2) |
| 19', 29': | Capturing Ligand (3) |

**Claims**

1. A method for detecting or quantifying an analyte, in which an analyte contained in a sample is detected or quantified by using a test strip for a lateral flow type chromatography which contains a membrane and a detection part on which a capturing ligand that is to specifically bind to the analyte has been fixed on the membrane, said method for detecting or quantifying an analyte comprising the following Steps (i) to (iii):

    Step (i) : a step of bringing an analyte contained in a sample into contact with a labeled ligand (1) wherein a ligand (1) that is to specifically bind to the analyte has been labeled with a phosphor (1) that is to be excited by a light having a wavelength of from 600 nm to 800 nm to generate a fluorescence,
    Step (ii): a step of bringing the complex (A), formed in Step (i) and containing the analyte and the labeled ligand, into contact with a capturing ligand at said detection part, and
    Step (iii) : a step of irradiating on the test strip a light having a wavelength of from 600 nm to 800 nm as an excitation light for the phosphor (1) contained in the complex (A) to generate a fluorescence from the phosphor (1), and measuring the fluorescence intensity of the fluorescence.

2. The method for detecting or quantifying an analyte according to claim 1, wherein the measurement wavelength of the fluorescence intensity in Step (iii) is not smaller than 600 nm.

3. The method for detecting or quantifying an analyte according to claim 1 or 2, wherein said said phosphor (1) is a fluorescent dye and/or a fluorescent protein.

4. The method for detecting or quantifying an analyte according to any one of claims 1 to 3, wherein, in the labeled ligand (1), said ligand (1) and said phosphor (1) have been held on an insoluble particle.

5. The method for detecting or quantifying an analyte according to any one of claims 1 to 4, wherein said insoluble particle is at least one selected from the group consisting of a synthetic polymer particle, an inorganic compound particle and a polysaccharide particle.

6. The method for detecting or quantifying an analyte according to any one of claims 1 to 4, wherein said insoluble particle has an average particle size of from 100 nm to 600 nm.

7. The method for detecting or quantifying an analyte according to any one of claims 1 to 6, which has a zeta potential of -30 mV or lower.

8. A kit for detecting or quantifying an analyte contained in a sample by using a test strip for a lateral flow type chromatography, said kit for detecting or quantifying an analyte comprising:

    a test strip for a lateral flow type chromatography which contains a membrane and a detection part on which a capturing. ligand that is to specifically bind to said analyte has been fixed on the membrane, and
    a detection reagent which contains a labeled ligand (1) wherein a ligand (1) that is to specifically bind to said analyte has been labeled with a phosphor (1) that is to be excited by a light having a wavelength of from 600 nm to 800 nm to generate a fluorescence.

9. A test strip for a lateral flow type chromatography for detecting or quantifying an analyte contained in a sample, said test strip for a lateral flow type chromatography comprising:

a membrane; and, on the membrane and in the direction that said sample develops,
a detection part on which a capturing ligand (2) that is to specifically bind to said analyte has been fixed, and, in a region upstream of the detection part, a reaction part which contains a labeled ligand (1) wherein a ligand (1) that is to specifically bind to said analyte has been labeled with a phosphor (1) that is to be excited by a light having a wavelength of from 600 nm to 800 nm to generate a fluorescence.

[Fig.1]

## INTERNATIONAL SEARCH REPORT

| | International application No. |
|---|---|
| | PCT/JP2013/060125 |

### A. CLASSIFICATION OF SUBJECT MATTER
*G01N33/543*(2006.01)i, *G01N21/64*(2006.01)i

According to International Patent Classification (IPC) or to both national classification and IPC

### B. FIELDS SEARCHED

Minimum documentation searched (classification system followed by classification symbols)
G01N33/543, G01N21/64

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2011-27693 A (Furukawa Electric Industrial Co., Ltd.), 10 February 2011 (10.02.2011), claims; paragraph [0041] (Family: none) | 1-9 |
| A | JP 2012-505402 A (Gyros Patent AB), 01 March 2012 (01.03.2012), claims; particularly, claim 10 & US 2011/0195524 A1 & EP 2344884 A & WO 2010/042031 A1 | 1-9 |

☐ Further documents are listed in the continuation of Box C.  ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 13 June, 2013 (13.06.13) | 25 June, 2013 (25.06.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

EP 2 835 643 A1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005522698 A
- JP 2011027693 A
- JP 2008304401 A
- JP 2009115822 A